# EUROPEAN PATENT SPECIFICATION

(11) **EP 0 674 649 B1**
(45) Date of publication and mention of the grant of the patent: **10.09.1997**
(21) Application number: 94903843.4
(22) Date of filing: 16.12.1993
(51) Int. Cl.: C07H 17/08, C07D 493/20, A61K 31/71, A01N 43/90, C12P 19/62, C12P 17/18, C12N 1/20

(54) **ANTIPARASITIC AGENTS**
ANTIPARASITISCHES MITTEL
AGENTS ANTIPARASITAIRES

(30) Priority: 19.12.1992 GB 9226525
(43) Date of publication of application: 04.10.1995
(73) Proprietor: Pfizer Limited, Sandwich Kent CT13 9NJ (GB); PFIZER INC., New York, N.Y. 10017 (US)
(72) Inventor: HAFNER, Edmund, William, Groton, CT 06340 (US); McARTHUR, Hamish, Alastair, Irvine, Groton, CT 06340 (US); PERRY, David, Austen, Groton, CT 06340 (US); PACEY, Michael, Stephen, Groton, CT 06340 (US); TYNAN, Edward, Joseph, Groton, CT 06340 (US)
(74) Representative: Moore, James William, Dr.
(86) International application number: EP9303625
(87) International publication number: WO9414830

(56) References cited:
- EP-A- 0 046 045
- EP-A- 0 205 251
- TETRAHEDRON LETTERS vol. 26, no. 36 , 1985 , OXFORD, GB pages 4283 - 4286 A. B. SMITH AND A. S. THOMPSON 'Avermectin-milbemycin studies. Synthesis of a milbemycin-avermectin hybrid.'
- TETRAHEDRON LETTERS vol. 26, no. 36, 1985, Oxford, GB, pp. 4283-4286, A.B. SMITH et al.

## Description

This invention relates to antiparasitic agents and in particular to compounds related to the avermectins but in which the oxygen atom bonded to the 6,8a positions of the avermectin nucleus is missing, that is the compounds are 6,8a-seco deoxyavermectin derivatives. The invention also relates to methods of making these compounds and to compositions containing them.

The avermectins are a group of broad spectrum antiparasitic agents referred to previously as the C-076 compounds. They are produced by fermenting a strain of the micro-organism Streptomyces avermitilis under aerobic conditions in an aqueous nutrient medium containing inorganic salts and assimilable sources of carbon and nitrogen. The isolation and the chemical structure of the eight individual components which make up the C-076 complex is described in detail in British Patent Specification 1573955.

In our European Patent Applications 0214731, 0284176, 0317148, 0308145, 0340832, 0335541 and 0350187 there are described preparations of compounds related to the avermectins but having a group at the 25-position other than the isopropyl or sec-butyl groups found in the original avermectin compounds disclosed in British Patent Specification 1573955. Such compounds may be prepared by fermentation of particular strains of Streptomyces avermitilis in the presence of organic acids or derivatives thereof.

It has now been discovered that certain mutants of these strains are capable of producing during fermentation compounds related to the avermectins but in which the oxygen atom bonded to the 6,8a positions of the avermectin nucleus is absent, i.e. they are 6,8a-seco-deoxyavermectins. The present invention relates to these compounds and to certain derivatives which may be made from them.

According to one aspect of the invention, there are provided compounds of formula (I), (II) or (III): 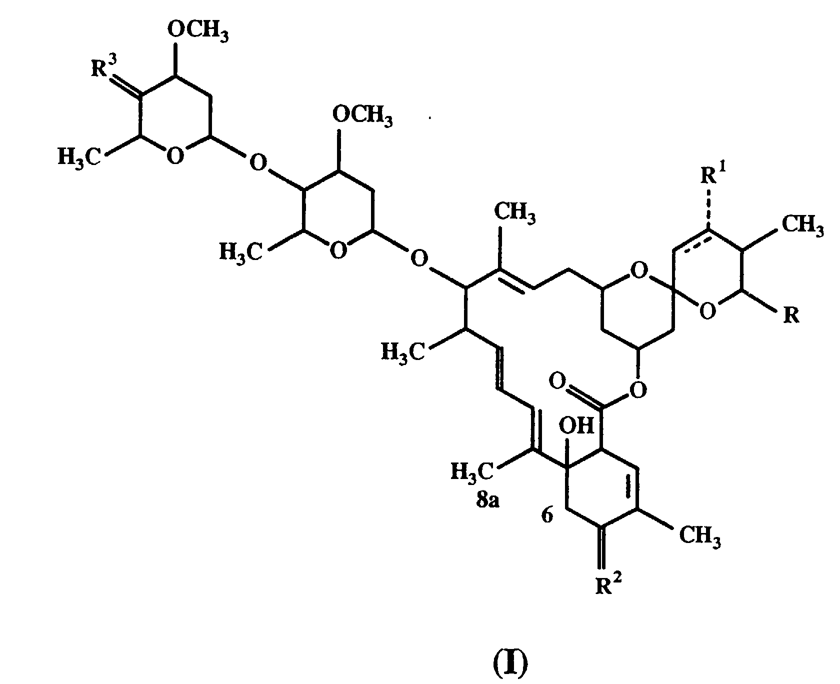 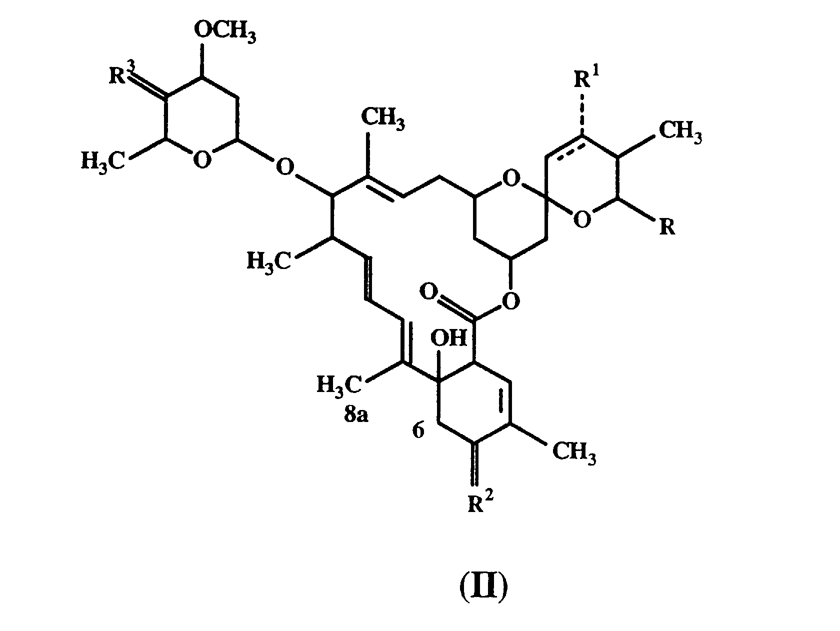 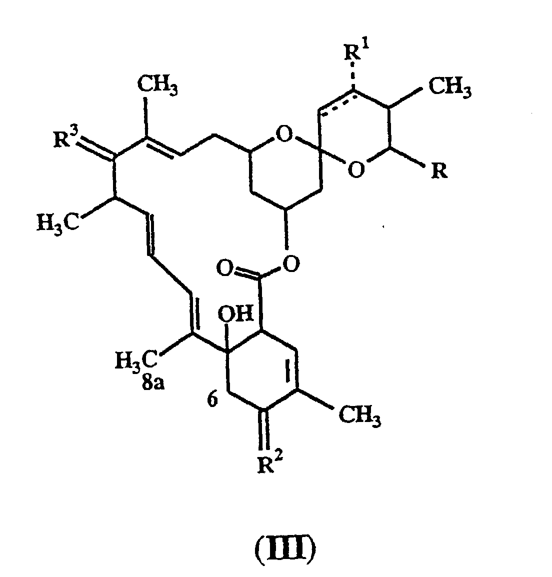 wherein R is:-
a C₃-C₈ cycloalkyl group which may optionally be substituted by methylene or one or more C₁-C₄ alkyl groups or halo atoms; or a 3 to 6 membered oxygen or sulphur containing heterocyclic ring which may be saturated, or fully or partially unsaturated and which may optionally be substituted by one or more C₁-C₄ alkyl groups or halo atoms;
the broken line at the 22-23 position represents an optional double bond, wherein R¹ is H, OH, O-(C₁-C₆ alkyl), oxo or optionally substituted oximino when the double bond is absent, or, the double bond is present and R¹ is absent;and R² and R³ are independently H and OH, H and C₁-C₆ alkoxy or H and C₁-C₆ acyloxy, these radicals being attached to the remainder of the molecule by single bonds, or are oxo or optionally O-substituted oximino attached by a double bond,
or in formula (I) or (II) R² is as defined above and R³ is H, amino wherein said amino is optionally substituted with a C₁-C₈ alkyl, aralkyl or acyl group or a C₃-C₈ alkenyl, or alkynyl group.

In the above definitions "halo" means F, Cl, Br or I.

U.S. Patent 4378353 mentions certain 6,8a-seco-deoxyavermectins which are made by fermentation using a mutant culture deposited under no. ATCC 31780. These compounds are of the formulae (IV), (V) and (VI): 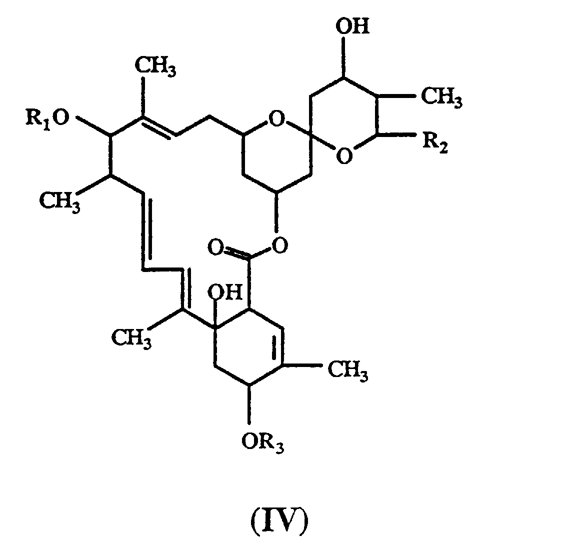 wherein
(a) R₁ is α-L-oleandrosyl; R₂ is sec-butyl; and R₃ is methyl;
(b) R₁ is α-L-oleandrosyl; R₂ is sec-butyl; and R₃ is hydrogen;
(c) R₁ is α-L-oleandrosyl-α-L-oleandrosyl; R₂ is sec-butyl and R₃ is hydrogen. 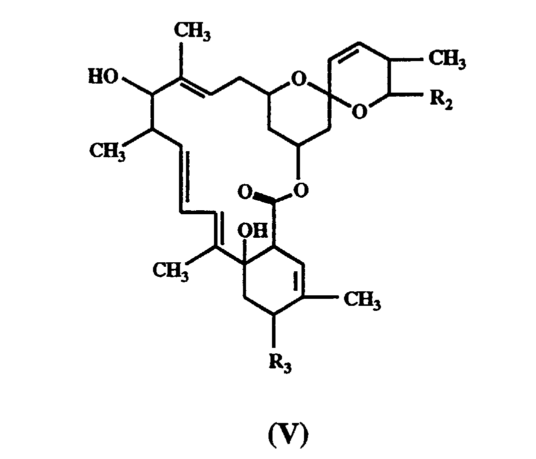
wherein
- (a) R₂ is iso-propyl; and R₃ is methoxy;
(b) R₂ is sec-butyl; and R₃ is keto;
(c) R₂ is iso-propyl; and R₃ is keto; and 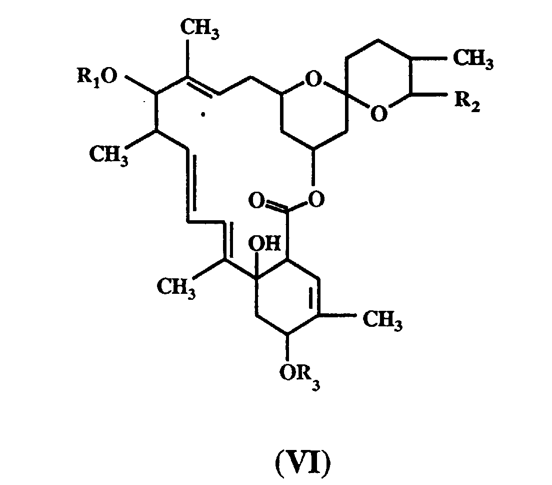
wherein R₁ is α-L-oleandrosyl-α-L-oleandrosyl; R₂ is sec-butyl; and R₃ is hydrogen.

U.S. Patent 4285963 mentions seco-avermectins produced by fermentation using the micro-organisms which produced the originally-discovered C-076 compounds. These compounds are of the formulae (VII) and (VIII): 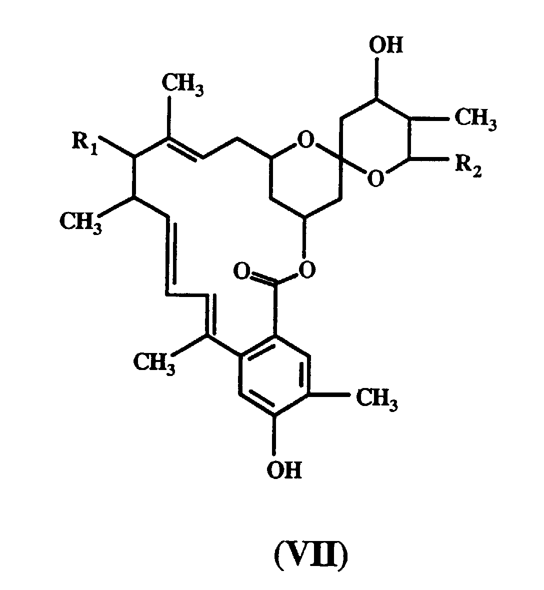 wherein
(a) R₁ is -OH and R₂ is sec-butyl;
(b) R₁ is =O and R₂ is sec-butyl;
(c) R₁ is -OH and R₂ is iso-propyl; and 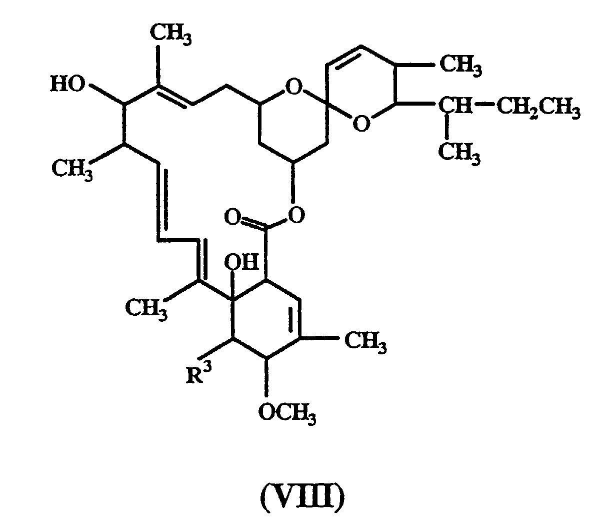
wherein R₃ is (a) hydrogen and (b) hydroxyl.

The R group of the 25- position in compounds (I), (II) at (III) is preferably a C₃-C₈ cycloalkyl group or a 5-or 6- membered oxygen- or sulphur- containing heterocyclic group. R is most preferably a cyclohexyl, cyclobutyl or 3 - thienyl group. In preferred compounds either the optional bond is present at the 22-23 position, or this optional bond is absent and R¹ is H or OH or oximino. R² is preferably H, OH or oxo or oximino. R³ is preferably H, OH or H, NHR⁸ where R⁸ is H, C₁-C₈ alkyl or acyl.
EP-A-205251 describes macrocyclic lactones related to the avermectins produced by fermentation of micro-organism ATCC-53116.

The C-076 compounds comprise eight distinct but closely related compounds described as C-076 A1a, A1b, A2a, A2b, B1a, B1b, B2a and B2b. The "a" series of C-076 compounds have an (S)-sec-butyl constituent at the 25-position of the avermectin nucleus and the "b" series have an isopropyl group at this position. The designations "A" and "B" refer to avermectins wherein the 5-substituent is methoxy or hydroxy, respectively, numeral "1" refers to avermectins in which a double bond is present at the 22-23 position and numeral "2" to avermectins lacking the 22-23 double bond and having a hydrogen at the 22-position and hydroxy at the 23-position. In this specification the "a" and "b" identifiers have been dropped but the identifiers A1, A2, B1 and B2 have been retained to refer to secodeoxyavermectins having the structural features corresponding to those of the original C-076 avermectins mentioned above.

Some of the compounds of the invention may be made by fermenting the strain of the micro-organism deposited at the American Type Culture Collection, 12301 Parklawn Drive, Rockville, Maryland 20852, USA under accession number ATCC 55372. This micro-organism is a Streptomyces avermitilis strain related to known Streptomyces avermitilis strain ATCC 31272, of which the morphological and cultural properties are described in British Patent Specification 1573955. The properties of ATCC 31272 and ATCC 55372 are compared and contrasted in following Tables 1, 2 and 3. Regarding antimicrobial activity, ATCC 31272 shows antibiosis against Streptomyces murinus 19788 and Aspergillus niger 16404, whereas ATCC 55372 does not.

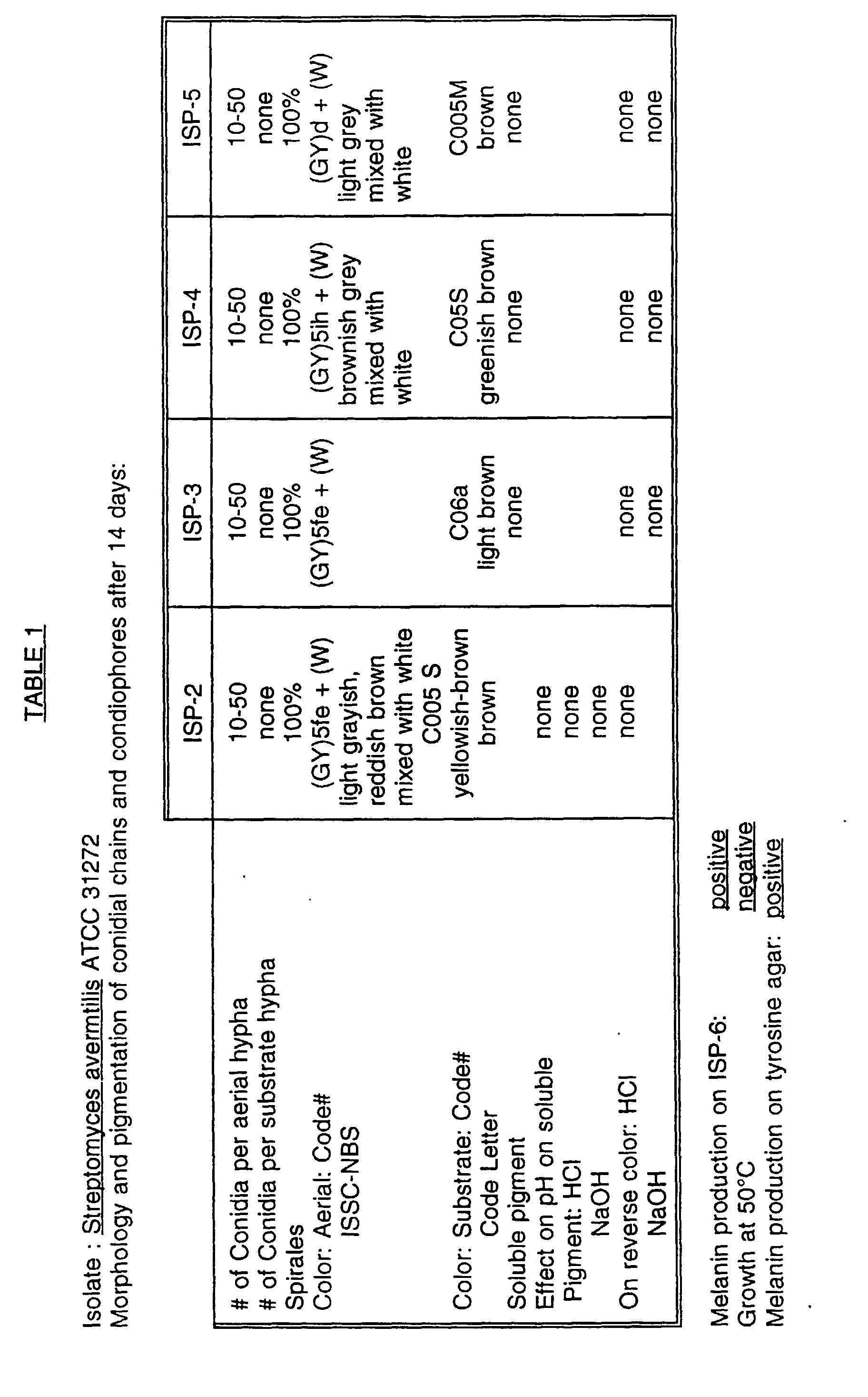

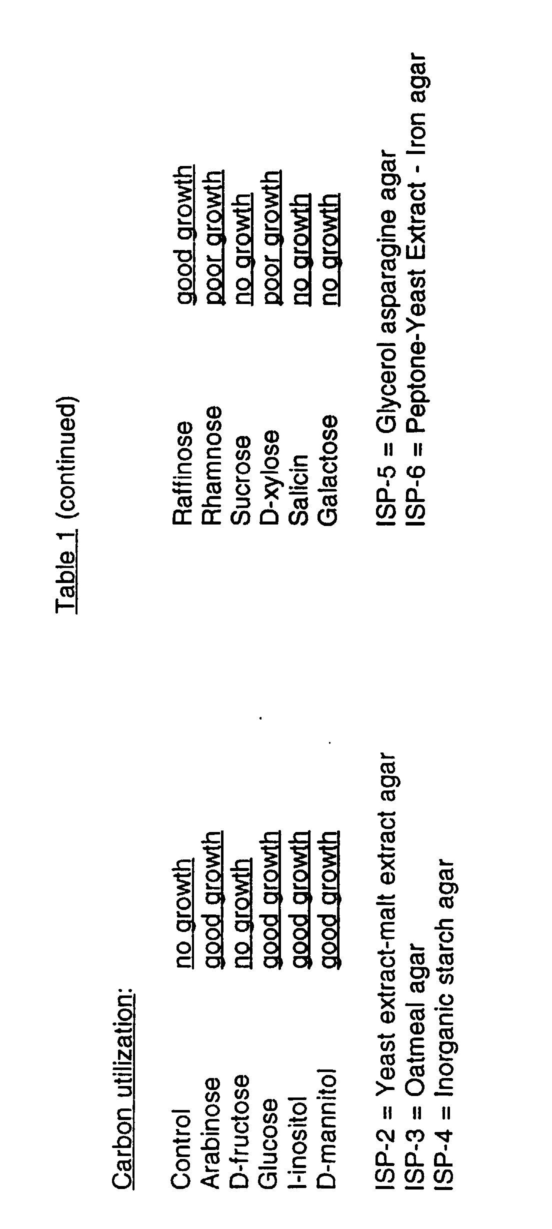

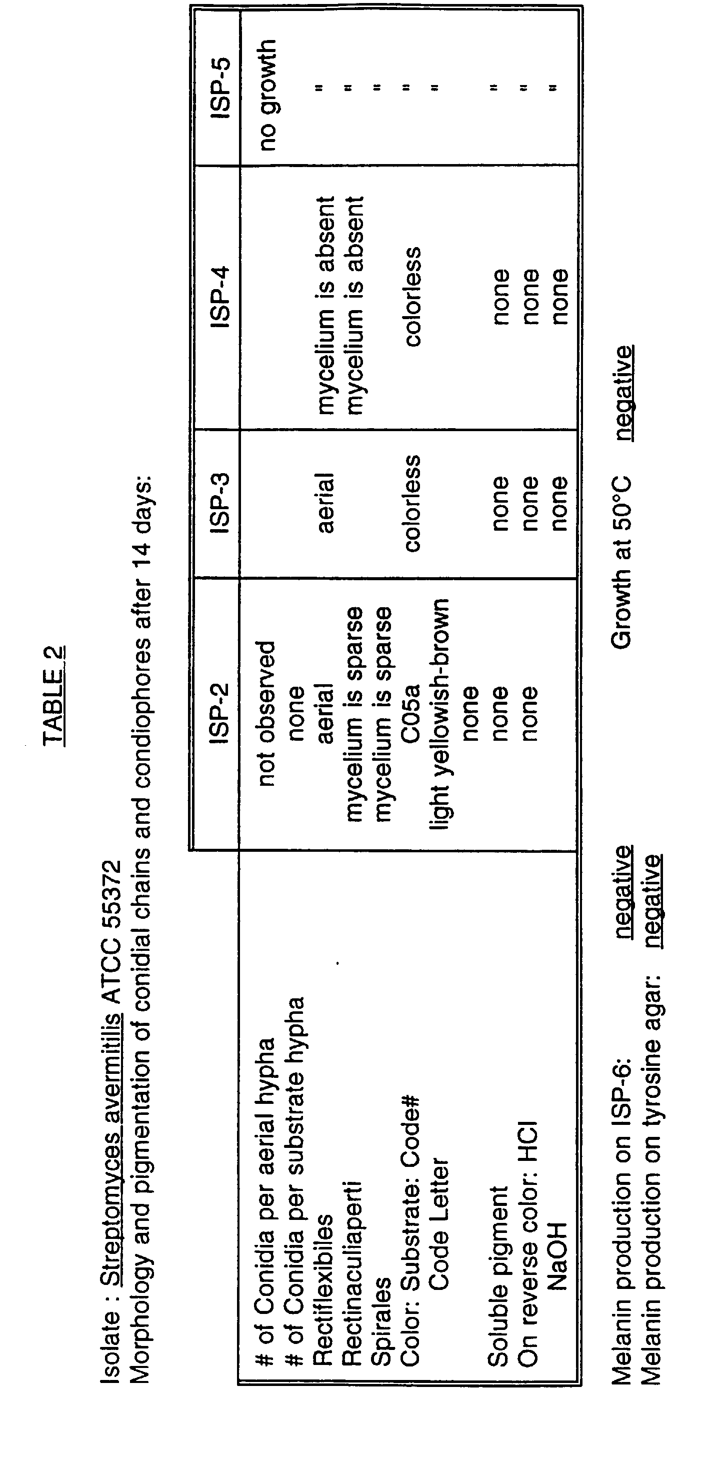

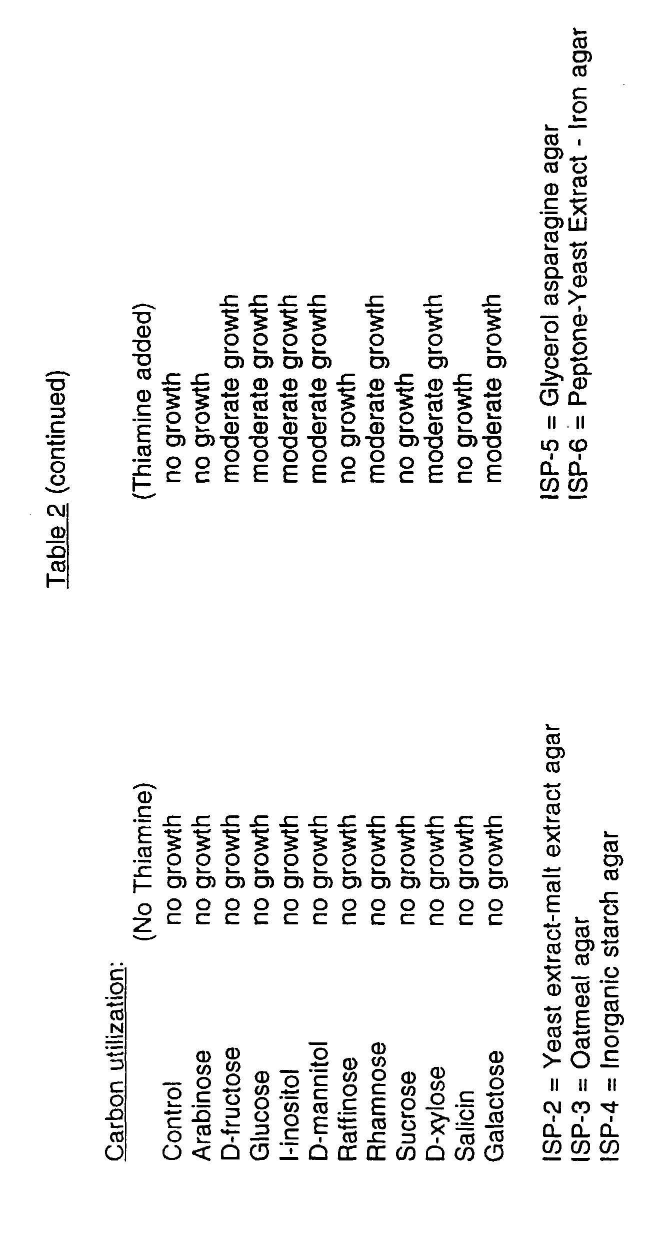

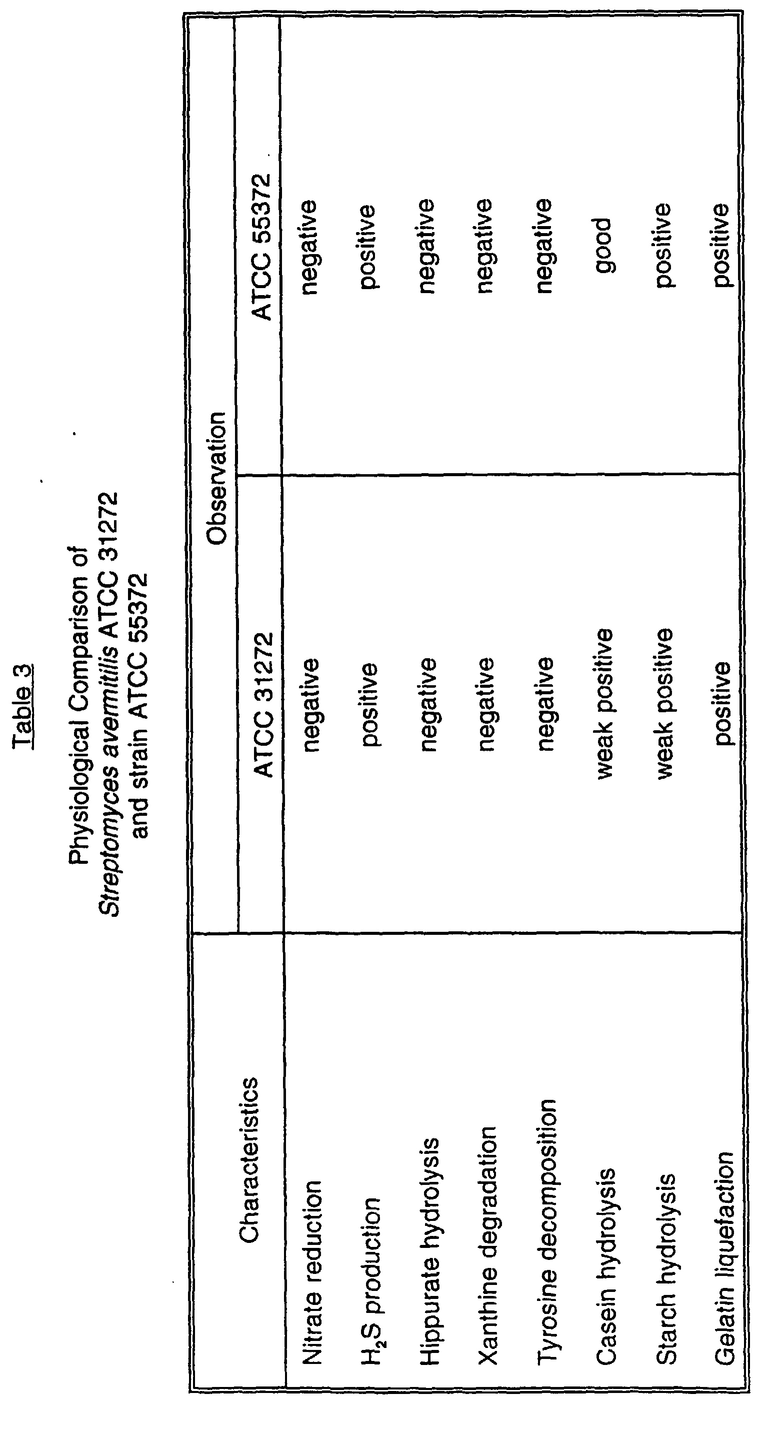

The fermentation should be conducted under aerobic conditions in an aqueous nutrient medium containing inorganic salts and assimilable sources of carbon and nitrogen. In the absence of an added carboxylic acid or suitable precursor no compounds of the invention are produced by the fermentation; compounds in which R is one of the groups identified above are obtained when a carboxylic acid of formula R-CO₂H or a salt, ester or amide thereof is added to the fermentation medium. The fermentation and isolation of the seco-deoxyavermectins obtained may be carried out as described in European Patent Application 0214731 et seq. mentioned above.

The compounds of the invention or the compounds convertible thereto obtained by fermentation in this way are: (i) compounds of formula I, that is the seco-deoxyavermectin disaccharide, in which R² and R³ are H, OH and either the double bond at the 22-23 position is present and R¹ is absent, or the double bond is absent and R¹ is OH, and (ii) the monosaccharide compounds of formula II in which R³ is H, OH, R² is either H, -OH or =O and either the double bond at the 22-23 position is present and R¹ is absent, or the double bond is absent and R¹ is OH.

Other compounds of the invention may be made by synthesis starting from these fermentation - derived seco-deoxyavermectins. The synthetic steps required to provide the desired compounds may require sequential reactions at different positions around the seco-deoxyavermectin nucleus and the exact order of these operations may vary. In practice certain functionalities in the molecule may be incompatible with the reaction conditions used and a protecting group is then required to avoid undesirable side reactions. A preferred protecting group for exposed hydroxy groups is tertbutyldimethylsilyl (TBDMS). Some of chemistry involved in these synthetic transformations is reviewed in Macrolide Antibiotics, Omura Sp., Ed., Academic Press, New York (1984) and by Davies, H.G. and Green, R.H. in Natural Product Reports (1986), 3, 87-121; Chemical Society Reviews (1991), 20, 211-269 and 271-339.

The disaccharide compounds may also generally be converted to the corresponding monosaccharide compounds, and the monosaccharides to the aglycones, by hydrolysis with dilute sulphuric acid in the presence of a solvent such as isopropyl alcohol.

The R² group may be converted from H, OH to H, OCH₃, when desired, by reaction with a methyl halide, preferably the iodide, in the presence of silver salts, preferably the oxide, by a method analagous to that described in United States patent 4,200,581.

The R² group may be converted from H, -OH to oxo, when desired, by reaction with manganese dioxide. The R¹ and R³ group may be converted from H, OH to oxo, when desired, by reaction with a suitable oxidizing agent for example using the Swern -procedure as described in United States patent 4427663. The corresponding oximino derivatives may be formed by reaction of the oxo compound with O-substituted or unsubstituted hydroxylamine, followed by O-acylation or alkylation if necessary.

The R₃ group may be converted from oxo to H, amino, when desired, by reductive amination according to the procedure described in United States patent 4,427,663.

Compounds in which R¹ is an alkoxy group may be prepared from the corresponding compound in which R¹ is OH by reaction with an appropriate alkyl bromide or iodide in the presence of a silver salt such as silver salicylate, as described in our copending European patent application 0623137.

When desired, the compounds of the invention having a double bond at the 22-23 position may be converted to the corresponding compounds having a single bond by hydrogenation in the presence of a suitable catalyst such as Wilkinson's catalyst as described in United States patent 4199569.

The compounds of the invention are effective in treating a variety of conditions caused by endoparasites including, in particular, helminthiasis which is most frequently caused by a group of parasitic worms described as nematodes and which can cause severe economic losses in swine, sheep, horses and cattle as well as affecting domestic animals and poultry. The compounds are also effective against other nematodes which affect various species of animals including, for example:- Dirofilaria in dogs and various parasites which can infect livestock, companion animals such as cats and dogs and also humans including gastro-intestinal parasites such as Ancylostoma, Necator, Ascaris, Strongyloides, Trichinella, Capillaria, Toxocara, Toxascaris, Trichuris, Enterobius and parasites which are found in the blood or other tissues and organs such as filarial worms and the extra intestinal stages of Strongyloides, Toxocara and Trichinella.

The compounds are also of particular value in treating ectoparasite infections including arthropod ectoparasites of animal and birds such as ticks, mites, lice, fleas, blowfly, biting insects and migrating dipterous larvae which can affect cattle and horses.

The compounds are also insecticides active against household pests such as the cockroach, clothes moth, carpet beetle and the housefly as well as being useful against arthropod pests of stored grain and of agricultural plants such as spider mites, aphids, caterpillars and against migratory orthopterans such as locusts.

Unexpectedly we have discovered that compounds within the scope of this invention have highly potent activity against important arthropod parasites.

The compounds of formula (I), (II) or (III) may be administered as a formulation appropriate to the specific use envisaged and to the particular species of host animal being treated and the parasite or insect involved. For use as an anthelmintic the compounds may be administered by injection, either subcutaneously or intramuscularly, altematively they may be administered orally in the form of a capsule, bolus, tablet, chewable tablet or liquid drench, or they may be administered as a pour-on or spot-on formulation or as an implant. Such formulations are prepared in a conventional manner in accordance with standard veterinary practice. Thus capsules, boluses or tablets may be prepared by mixing the active ingredient with a suitable finely divided diluent or carrier, additionally containing a disintegrating agent and/or binder such as starch, lactose, talc, or magnesium stearate. A drench formulation may be prepared by dispersing the active ingredient in an aqueous solution together with dispersing or wetting agents and injectable formulations may be prepared in the form of a sterile solution or emulsion. Pour-on or spot-on formulations may be prepared by dissolving the active ingredient in an acceptable liquid carrier vehicle, such as butyl digol, liquid paraffin or non-volatile ester with or without addition of a volatile component such as isopropanol. These formulations will vary with regard to the weight of active compound depending on the species of host animal to be treated, the severity and type of infection and the body weight of the host. Generally for oral, parenteral and pour-on administration a dose of from about 0.001 to 10mg per kg of animal body weight given as a single dose or in divided doses for a period of from 1 to 5 days will be satisfactory but of course there can be instances where higher or lower dosage ranges are indicated and such are within the scope of this invention.

As an alternative the compounds may be administered with the animal feedstuff and for this purpose a concentrated feed additive or premix may be prepared for mixing with the normal animal feed.

For use as an insecticide and for treating agricultural pests the compounds are applied as sprays, dusts, pour-on or spot-on formulations, emulsions and the like in accordance with standard agricultural practice.

For human use the compounds are administered as a pharmaceutically acceptable formulation in accordance with normal medical practice.

The preparation of compounds according to the invention are illustrated by the following Examples.

### Example 1

A frozen inoculum (2ml) of a culture of Streptomyces avermitilis mutant strain ATCC 55372 was inoculated into 50ml of a medium containing starch (1g), Pharmamedia (Trademark) (0.75g), Ardamine pH (Trademark) (0.25g) and calcium carbonate (0.1g) in a 300ml flask and incubated at 28°C for 2 days. This inoculum (50ml) was transferred to a second inoculum flask (1 litre) containing starch (20g), Pharmamedia (15g), Ardamine pH (5g) and calcium carbonate (2g) and incubated at 28°C for a further 2 days. This inoculum was added to 100 litres of a medium containing starch (8kg), Oxoid (Trademark) yeast extract (500g), magnesium sulphate heptahydrate (100g), dipotassium hydrogen phosphate (100g), monosodium glutamate (100g), zinc sulphate (0.1g), manganese (II) chloride (0.1g), iron (II) sulphate (10g) and calcium carbonate (700g) contained in a 150 litre fermenter and with a natural pH of 7.05. The fermentation was incubated at 29°C with agitation at 200 rpm and with aeration at 40 litres per minute. Cyclohexanecarboxylic acid sodium salt (200g) was added to the fermentation after 24 hours. After 14 days the broth was harvested by ethyl acetate extraction and the organic layer was filtered and concentrated to an oil (62g) which was then chromatographed on 1kg silica (Merck kieselgel 60) eluting initially with dichloromethane then dichloromethane containing a progressively higher proportion of ethyl acetate. The desired compounds began to elute when the mobile phase was 4:1 dichloromethane:ethyl acetate. These were recognised by their characteristic high performance liquid chromatography retention times and ultraviolet spectra, as captured by a diode array detector, as shown in the Table 4 below.

**Table 4**

| Retention | | |
|---|---|---|
| Compound | Time, min, * | UV, max nm |
| Seco-6,8a-deoxy-25-cyclohexylavermectin B1 | 30.3 | 237(sh),241 |
| Seco-6,8a-deoxy-25-cyclohexylavermectin B2 | 20.9 | 237(sh),241 |
| Seco-6,8a-deoxy-25-cyclohexylavermectin B1 monosaccharide | 23.4 | 237(sh),241 |
| Seco-6,8a-deoxy-25-cyclohexylavermectin B2 monosaccharide | 14.9 | 237(sh),241 |
| 5-Keto-seco-6,8a-deoxy-25-cyclohexylavermectin B1 monosaccharide | 24.0 | 237 |
| 5-Keto-seco-6,8a-deoxy-25-cyclohexylavermectin B2 monosaccharide | 15.3 | 237 |

| | | |
|---|---|---|
| * Beckman Ultrasphere ODS (Trademark) 5µm, 4.6x250mm, eluting with a methanol-water gradient of 80:20 linearly to 95:5 over 40 min at 0.85ml per minute and 40°C. | | |

Final purification of the desired compounds was achieved by a combination of reverse phase high performance liquid chromatography and normal phase (silica) column chromatography. The NMR spectra obtained were as follows.

Seco-6,8a-deoxy-25-cyclohexylavermectin B1. NMR(CDCl₃)(in part): δ = 6.3(d), 6.05(dd), 5.75(dd), 5.65(dd), 5.55(dd), 3.5(s), 3.45(s), 3.3(t), 3.2(t), 1.85(bs), 1.75(bs).

Seco-6,8a-deoxy-25-cyclohexylavermectin B2. NMR (CDCl₃) (in part): δ = 6.3(d), 6.05(dd), 5.65(dd), 3.5(s), 3.45(s), 3.25(t), 3.20(t), 1.9(bs), 1.75(bs). Seco-6,8a-deoxy-25-cyclohexylavermectin B1 monosaccaride. NMR (CDCl₃) (in part): δ = 6.3(d), 6.0(dd), 5.75(dd), 5.6(dd), 5.55(dd), 3.5(s), 3.15(t), 1.85(bs), 1.70(bs).

Seco-6,8a-deoxy-25-cyclohexylavermectin B2 monosaccharide. NMR (CDCl₃) (in part): δ = 6.25(d), 6.0(dd), 5.6(dd), 5.3(m), 3.5(s), 3.15(t), 1.85(bs) 1.70(bs).

5-Keto-seco-6,8a-deoxy-25-cyclohexylavermectin B1 monosaccharide. NMR (CDCl₃) (in part): δ = 6.42(bs), 6.3(d), 6.0(dd), 5.75(dd), 5.6(dd), 5.55(dd), 3.5(s), 3.15(t), 2.8(dd), 1.85(bs), 1.75(bs).

5-Keto-seco-6,8a-deoxy-25-cyclohexylavermectin B2 monosaccharide. NMR (CDCl₃) (in part): δ = 6.45(bs), 6.3(d), 6.0(dd), 5.6(dd), 5.35(m), 3.5(s), 3.15(t), 2.8(dd), 1.85(bs), 1.75(bs).

### Example 2

### Seco-6,8a-deoxy-25-cyclohexylavermectin B2 monosaccharide

Seco-6,8a-deoxy-25-cyclohexylavermectin B2 (1.0g) (Example 1) was dissolved in isopropanol (10ml) and a solution of sulphuric acid (0.2ml) in isopropanol (10ml) was added. The solution was stirred at room temperature for 20 hours then poured into water, extracted -with methylene chloride and the layers separated. The organic layer was dried over anhydrous sodium sulphate and evaporated to dryness. The crude product was chromatographed on 8µm SiO₂-CDS (40.1 x 250mm, Dynamax, Trademark-Rainin) eluting with methanol-water 4:1 at 69ml per minute to give the title compound (618mg) identical to material isolated from the fermentation of Example 1 and Seco-6,8a-deoxy-25-cyclohexylavermectin B2 aglycone (33mg). NMR (CDCl₃) (in part) δ = 6.25(d), 6.05(dd), 5.55(dd), 5.3(m), 5.15(m), 4.45(m), 3.95(bs), 3.55(d), 3.35(d), 1.85(bs), 1.70(bs), 1.60(bs), 0.9(d), 0.75(q).

### Example 3

### Seco-6,8a-deoxy-22,23-dihydro-25-cyclohexylavermectin B1

Seco-6,8a-deoxy-25-cyclohexylavermectin B1 (1.3g) (Example 1) was dissolved in degassed toluene (200ml) and tris(triphenylphosphine)rhodium (I) chloride (100mg) was added. After a brief period of sonication the solution was shaken under a hydrogen atmosphere (35-70KPa) at room temperature. After 48 hours a further 100mg of catalyst was added, the hydrogen pressure raised to 280KPa and the temperature raised to 35°C. After 20 hours the solution was filtered through silica, washed through with methanol and the combined solutions evaporated to dryness. The title compound was finally purified by reverse phase hplc eluting with methanol-water 95:5. Both NMR and mass spectroscopic data were fully consistent with the proposed structure.

### Example 4

### Seco-6,8a-deoxy-22,23-dihydro-25-cyclohexylavermectin B1 monosaccharide

The title compound was prepared from the product of Example 3 by a method exactly similar to that described in Example 2. NMR and mass spectroscopic data were fully consistent with the proposed structure.

### Example 5

### 5-Oximino-seco-6,8a-deoxy-22,23-dihydro-25-cyclohexylavermectin B1

6,8a-Deoxy-22,23-dihydro-25-cyclohexylavermectin B1 (50mg) (Example 3) was dissolved in ether (50ml) and manganese (IV) oxide (100mg) was added. The suspension was stirred at room temperature, filtered and evaporated to dryness to give 5-keto-6,8a-deoxy-22,23-dihydro-25-cyclohexylavermectin B1 as a white solid (40mg). This was dissolved in pyridine (2ml) and hydroxylamine hydrochloride (50mg) was added. The mixture was stirred at room temperature for 24 hours then poured into dilute hydrochloric acid, extracted with methylene chloride and the organic layer was dried over anhydrous sodium sulphate and evaporated to dryness. The residue was purified by reverse phase hplc eluting with methanol-water 95:5 to give the pure title compound (28mg) as a white solid. NMR and mass spectroscopic data were fully consistent with the proposed structure.

### Example 6

### 5-Oximino-seco-6,8a-deoxy-22,23-dihydro-25-cydohexylavermectin B1 monosaccharide

6,8a-Deoxy-22,23-dihydro-25-cyclohexylavermectin B1 monosaccharide (Example 4) was converted to the title compound by a method exactly similar to that described in Example 5. NMR and mass spectroscopic data were fully consistent with the proposed structure.

### Example 7

### 5-Oximino-seco-6,8a-25-cyclohexylavermectin B1 monosaccharide

5-Keto-seco-6,8a-deoxy-25-cyclohexylavermectin B1 monosaccharide (77mg) (Example 1) was dissolved in pyridine (2ml) and hydroxylamine hydrochloride (100mg) was added. The mixture was stirred overnight at room temperature for 18 hours then poured into an ice/water mixture, acidified by adding dilute sulphuric acid (2N) and extracted with methylene chloride. The organic extract was dried over anhydrous sodium sulphate and evaporated to dryness under reduced pressure. The residue was purified by reverse phase HPLC on a Dynamax column (24mm x 250cm, 5µm, ODS-silica, Rainin) eluting with methanol - water 90:10 at 9ml per minute. Fractions eluting between 17 and 23 minutes were combined and evaporated to dryness under vacuum to yield the title compound as a white powder (58mg). Mass and NMR spectroscopic data were fully consistent with the proposed structure.

### Example 8

### 5-Oximino-seco-6,8a-deoxy-25-cyclohexylavermectin B2 monosaccharide

5-Keto-seco-6,8a-deoxy-25-cyclohexylavermectin B2 monosaccharide (280mg) (Example 1) was treated with hydroxylamine hydrochloride (300mg) in pyridine (10ml) according to the method described in Example 7. Final purification was achieved using preparative reverse phase HPLC on a Dynamax column (24mm x 250cm, 5µm, ODS-silica, Rainin) eluting with methanol - water 85:15 at 9ml per minute. Appropriate fractions were combined and evaporated to dryness under vacuum to yield the title compound as a white powder (192mg). Mass and NMR spectroscopic data were fully consistent with the proposed structure.

### Example 9

Seco-6.8a-deoxy-25-cyclobutylavermectins were produced by adding cyclobutane carboxylic acid to a fermentation of the Streptomyces avermitilis mutant strain ATCC 55372 in a manner as described in Example 1 except that 10g of cyclobutane carboxylic acid was added after 43 hours and again after 68 hours and the whole fermentation harvested after 7 days. The desired compounds were recognised by their characteristic HPLC retention times and UV spectra, as captured by a diode array detector, as shown in Table 5 below.

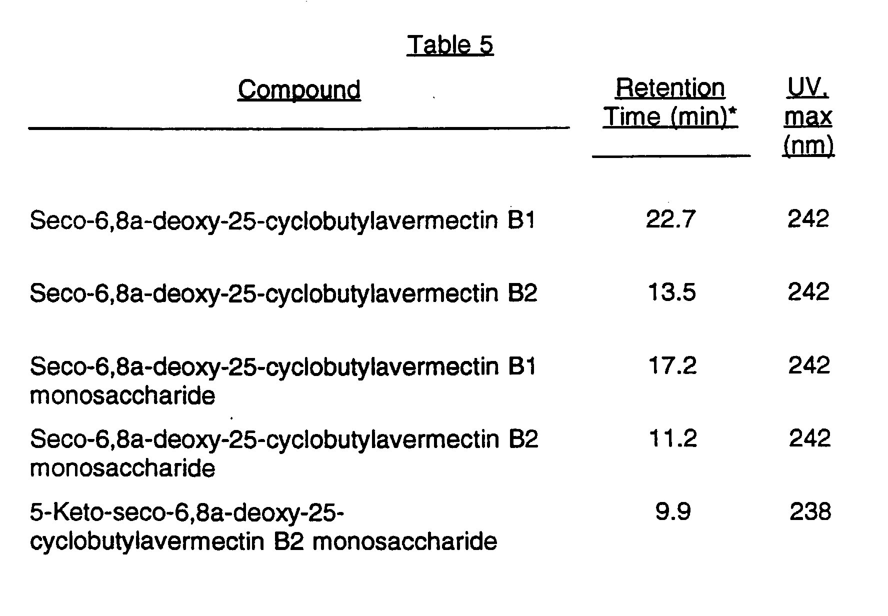

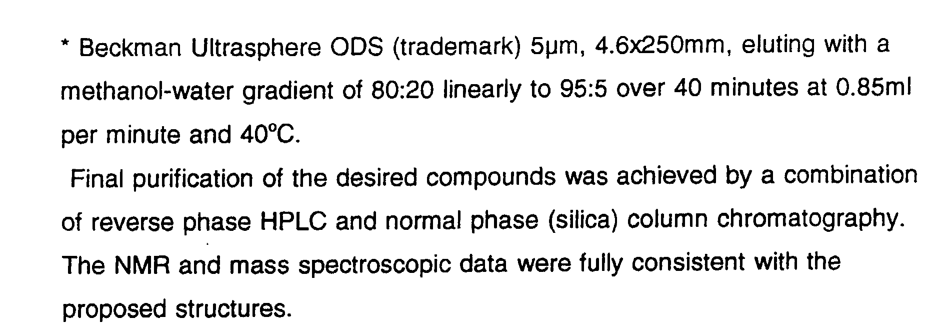

### Example 10

### Seco-6,8a-deoxy-25-cyclobutylavermectin B2 monosaccharide

Seco-6,8a-deoxy-25-cyclobutylavermectin B2 (0.5g) (Example 9) was dissolved in isopropyl alcohol (10ml) containing 1% sulphuric acid v/v. The solution was allowed to stand for 24 hours then poured into an ice/water mixture and extracted with methylene chloride (2x 50ml). The combined extracts were washed with a 5% w/v sodium bicarbonate solution, dried over anhydrous sodium sulphate and evaporated to dryness under reduced pressure to yield a crude product. Final purification was achieved using preparative reverse phase HPLC on a Dynamax column (24mm x 250cm, 5m, ODS-silica, Rainin) eluting with methanol - water 85:15 at 9ml per minute. Appropriate fractions were combined and evaporated to dryness under vacuum to yield the title compound as a white powder. Mass and NMR spectroscopic data were fully consistent with the proposed structure.

### Example 11

### Seco-6,8a-deoxy-22,23-dihydro-25-cyclobutylavermectin B1

Seco-6,8a-deoxy-25-cyclobutylavermectin B1 (0.8g) (Example 9) was dissolved in degassed toluene (200ml) and tris(triphenyl)phosphine) rhodium (I) chloride (1g) added. After a brief period of sonication hydrogen gas was slowly bubbled through the solution. After 20 hours the reaction mixture was filtered through silica, washed with methanol and the combined eluates evaporated to dryness to give a crude product which was further purified using preparative reverse phase HPLC on a Dynamax column (24mm x 250cm, 5µm, ODS-silica, Rainin) eluting with methanol - water 95:5 at 27ml per minute. Appropriate fractions were combined and evaporated to dryness under vacuum to yield the title compound as a white powder. Mass and NMR spectroscopic data were fully consistent with the proposed structure.

### Example 12

### Seco-6,8a-deoxy-22,23-dihydro-25-cyclobutylavermectin B1 monosaccharide

Seco-6,8a-deoxy-22,23-dihydro-25-cyclobutylavermectin B1 (0.48g) (Example 11) was dissolved in isopropyl alcohol (10ml) containing 1% sulphuric acid v/v. The desired product was recovered and purified using the method described in Example 10 except that the HPLC eluate used was methanol - water 90:10. Appropriate fractions were combined and evaporated to dryness under vacuum to yield the title compound as a white powder (266mg). Mass and NMR spectroscopic data were fully consistent with the proposed structure.

### Example 13

Seco-6,8a-deoxy-25-(3-thienyl)avermectins were produced by adding thiophene-3-carboxylic acid to a fermentation of the Streptomyces avermitilis mutant strain ATCC 55372 in a manner as described in Example 1 except that 10g thiophene-3-carboxylic acid was added after 43 hours and again after 68 hours and the whole fermentation harvested after 7 days. The desired compounds were recognised by their characteristic HPLC retention times and UV spectra, as captured by a diode array detector, as shown in Table 6 below.

**Table 6**

| Compound | Retention Time (min)* | UV, max (nm) |
|---|---|---|
| Seco-6,8a-deoxy-25-(3-thienyl)avermectin B1 | 18.1 | 240 |
| Seco-6,8a-deoxy-25-(3-thienyl)avermectin B2 | 10.0 | 240 |
| Seco-6,8a-deoxy-25-(3-thienyl)avermectin B1monosaccharide | 14.7 | 240 |
| Seco-6,8a-deoxy-25-(3-thienyl)avermectin B2 monosaccharide | 7.0 | 240 |

| | | |
|---|---|---|
| * Beckman Ultrasphere ODS (trademark) 5µm, 4.6x250mm, eluting with a methanol-water gradient of 80:20 linearly to 95:5 over 40 minutes at 0.85ml per minute and 40°C. | | |

Final purification of the desired compounds was achieved by a combination of reverse phase HPLC and normal phase (silica) column chromatography. The NMR and mass spectroscopic data were fully consistent with the proposed structures.

### Example 14

### Seco-6,8a-deoxy-25-(3-thienyl)avermectin B2 monosaccharide

Seco-6,8a-deoxy-25-(3-thienyl)avermectin B2 (0.3g) (Example 13) was dissolved in isopropyl alcohol (15ml) containing 1% sulphuric acid v/v. The desired product was recovered and purified using the method described in Example 10 except that the HPLC eluate used was methanol - water 80:20. Appropriate fractions were combined and evaporated to dryness under vacuum to yield the title compound as a white powder (180mg). Mass and NMR spectroscopic data were fully consistent with the proposed structure.

### Example 15

### 5-Oximino-seco-6,8a-deoxy-25-(3-thienyl)avermectin B2 monosaccharide

Seco-6,8a-deoxy-25-(3-thienyl)avermectin B2 monosaccharide (115mg) (Example 14) was oxidised with manganese (iv) oxide (0.2g) in diethyl ether (100ml) using the method described in Example 5 to give 5-keto-seco--6,8a-deoxy-25-(3-thienyl)avermectin B2 monosaccharide (90mg). This material was further reacted with hydroxylamine hydrochloride (100mg) in pyridine (4ml) again using the method described in Example 5 to yield the title compound as a white powder (75mg). Mass and NMR spectroscopic data were fully consistent with the proposed structure.

### Example 16

### Seco-6,8a-deoxy-22,23-dihydro-25-(3-thienyl)avermectin B1

Seco-6,8a-deoxy-25-(3-thienyl)avermectin B1 (50mg) (Example 13) was hydrogenated using tris(triphenyl)phosphine) rhodium (I) chloride (50mg) in degassed toluene (25ml) using the procedures described in Example 11 except that the final preparative HPLC purification used an eluate consisting of methanol - water 90:10. The title compound was obtained as a white powder (25mg). Mass and NMR spectroscopic data were fully consistent with the proposed structure.

### Example 17

### Seco-6,8a-deoxy-22,23-dihydro-25-(3-thienyl)avermectin B1 monosaccharide

Seco-6,8a-deoxy-22,23-dihydro-25-(3-thienyl)avermectin B1 (17.5mg) (Example 16) was dissolved in isopropyl alcohol (0.5ml) containing 1% sulphuric acid v/v. The desired product was recovered and purified using the method described in Example 10 except that the final purification was achieved using preparative reverse phase HPLC on a Dynamax column (12mm x 250cm, 5µm, ODS-silica, Rainin) eluting with methanol - water 90:10 at 4ml per minute. Appropriate fractions were combined and evaporated to dryness under vacuum to yield the title compound as a white powder (7.5mg). Mass and NMR spectroscopic data were fully consistent with the proposed structure.

## Claims

1. A compound of formula (I), (II) or (III): 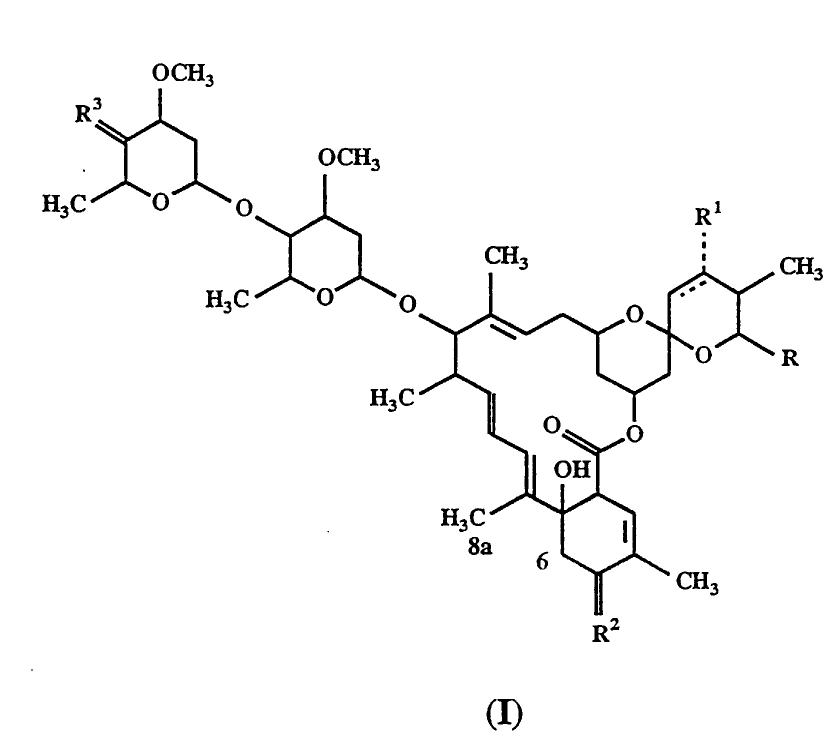 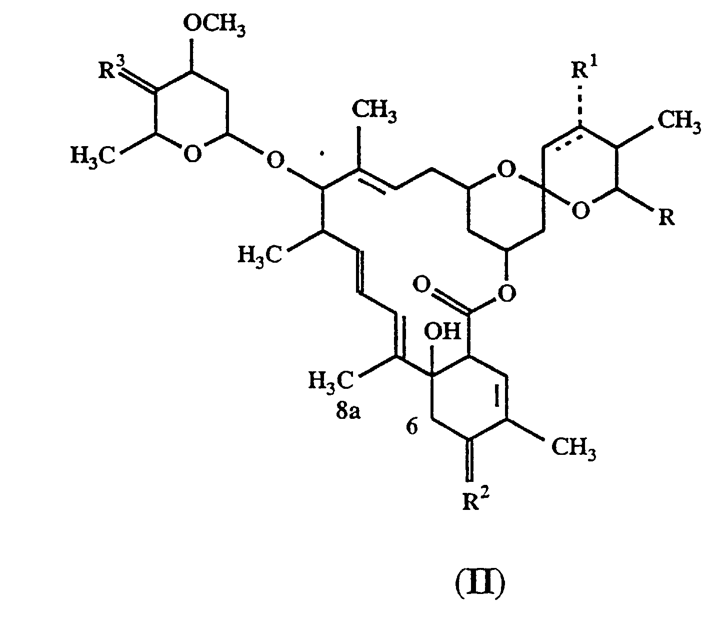 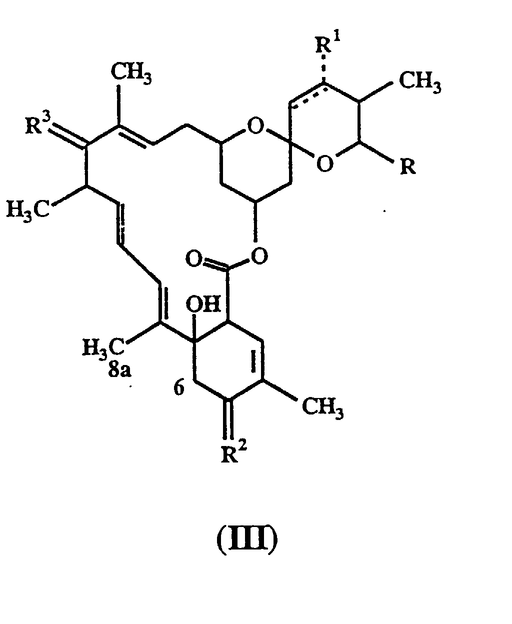 wherein R is:-
a C₃-C₈ cycloalkyl group which may optionally be substituted by methylene or one or more C₁-C₄ alkyl groups or halo atoms; or a 3 to 6 membered oxygen or sulphur containing heterocyclic ring which may be saturated, or fully or partially unsaturated and which may optionally be substituted by one or more C₁-C₄ alkyl groups or halo atoms;
the broken line at the 22-23 position represents an optional double bond, wherein R¹ is H, OH, O-(C₁-C₆ alkyl) oxo or optionally substituted oximino when the double bond is absent, or, the double bond is present and R¹ is absent; R² is H and OH, H and C₁-C₆ alkoxy or H and C₁-C₆ acyloxy, these radicals being attached to the remainder of the molecule by single bonds, or is oxo or optionally O - substituted oximino attached by a double bond; R³ is H and OH, H and C₁-C₆ alkoxy or H and C₁-C₆ acyloxy, these radicals being attached to the remainder of the molecule by single bonds, or is oxo or optionally O - substituted oximino attached by a double bond;
or in formula (I) or (II) R² is as defined above and R³ is H, amino wherein said amino is optionally substituted with a C₁-C₈ alkyl, aralkyl or acyl group or a C₃-C₈ alkenyl, or alkynyl group.

2. A compound according to claim 1, in which R is a C₃-C₈ cycloalkyl group or a 5 - or 6 - membered oxygen - or sulphur - containing heterocyclic group.

3. A compound according to claim 2 in which R is a cyclohexyl, cyclobutyl or 3- thienyl group.

4. A compound according to claim 1, 2 or 3 in which said optional bond is present or said optional bond is absent and R¹ is H or OH or oximino.

5. A compound according to any preceding claim, in which R² is H, OH, oxo or oximino.

6. A compound according to any preceding claim, in which R³ is H, OH or H, NHR⁸ where R⁸ is H, C₁-C₈ alkyl or acyl.

7. A process for preparing a compound according to any one of claims 1 to 6 or a compound convertible thereto, which comprises fermenting micro-organism Streptomyces avermitilis ATCC 55372 in the presence of a carboxylic acid of formula RCO₂H or a salt, ester or amide thereof where R is as defined in claim 1 to produce (a) a compound of formula (I) in which R² and R³ are both H, OH and either the double bond at the 22-23 position is present and R¹ is absent, or the double is absent and R¹ is OH; or (b) the compound of formula (II), in which R² is either H, OH or =O, R³ is H, OH and either the double bond at the 22-23 position is present and R¹ is absent, or the double bond is absent and R¹ is OH and isolating the compound of formula (I) or (II) produced; if necessary followed by one or more of the following steps:
(i) hydrolysing a compound of formula (I) to yield a compound of formula (II) or (III), or hydrolysing a compound of formula (II) to yield a compound of formula (III);
(ii) converting H, OH at R² to H, OCH₃ in a compound of formula (I), (II) or (III) by reaction thereof with a methyl halide in the presence of silver oxide or a silver salt;
(iii) oxidising a compound of formula (I), (II) or (III) in which R² or R³ is H, OH and/or R¹ is OH to yield a compound in which at least one of R¹ and R² is oxo;
(iv) allowing a compound obtained from (iii) to react with optionally O-substituted hydroxylamine to produce an optionally substituted imino compound;
(v) converting an oxo group at R³ of a compound obtained from (III) to a compound of formula (I), (II) or (III) to amino by reductive amination;
(vi) reacting a compound in which R¹ is OH with an alkyl bromide or iodide in the presence of silver oxide or a silver salt to produce a compound in which R¹ is -O-alkyl;
(vii) reducing a compound of formula (I), (II) or (III) having a double bond at the 22-23 position with hydrogen to yield a compound having a single bond at the 22-23 position.

8. A compound according to any one of claims 1 to 6, for use in human or veterinary medicine.

9. Use of a compound according to any one of claims 1 to 6, in the manufacture of a medicament for treatment or prophylaxis of parasitic infections.

10. Streptomyces avermitilis, ATCC 55372.

## Patentansprüche

1. Verbindung der Formel (I), (II) oder (III): 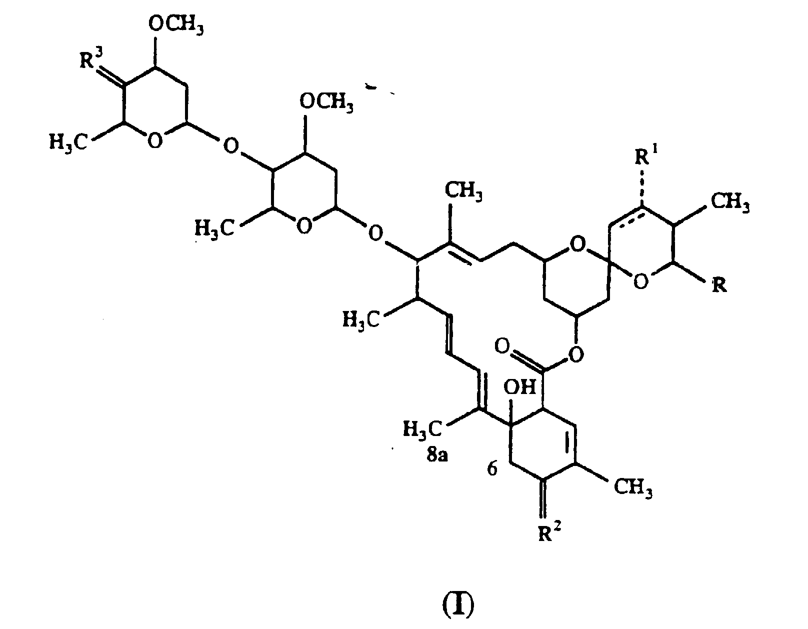 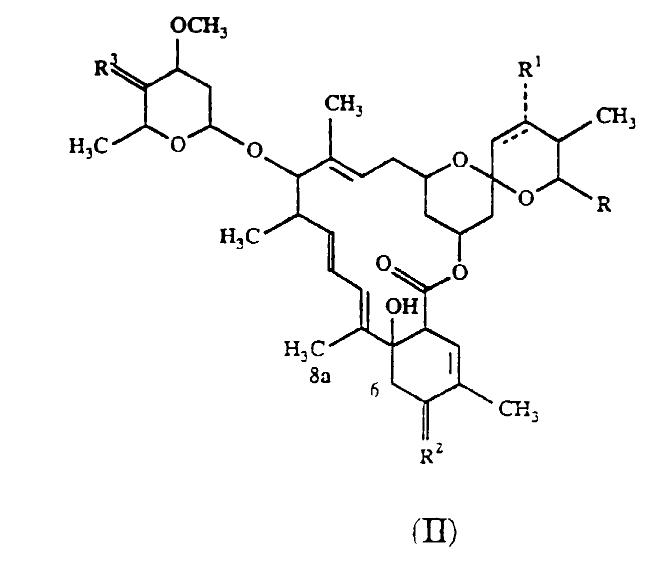 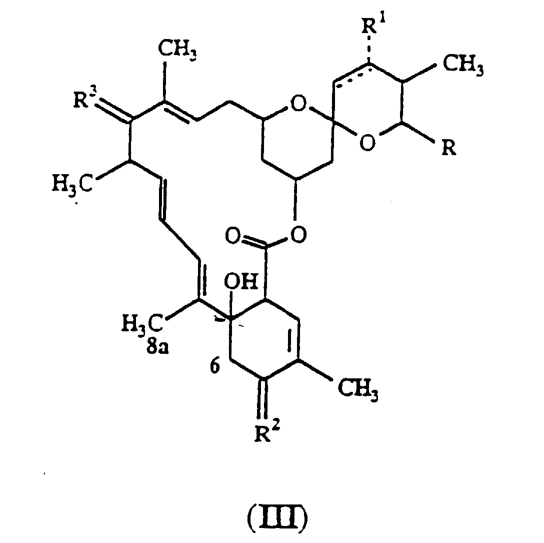 worin R:
eine C₃-C₈-Cycloalkylgruppe, die gegebenenfalls mit Methylen oder einer oder mehreren C₁-C₄-Alkylgruppen oder Halogenatomen substituiert sein kann, oder einen 3- bis 6- gliedrigen, Sauerstoff- oder Schwefel-enthaltenden heterocyclischen Ring darstellt, der gesättigt oder vollständig oder teilweise ungesättigt sein kann und der mit einer oder mehreren C₁-C₄-Alkylgruppen oder Halogenatomen gegebenenfalls substituiert sein kann, wobei die gestrichelte Linie der 22-23-Stellung eine gegebenenfalls vorliegende Doppelbindung wiedergibt, worin R¹ H, OH, O-(C₁-C₆-Alkyl), Oxo oder eine gegebenenfalls substituierte Oximinogruppe darstellt, wenn die Doppelbindung nicht vorliegt oder die Doppelbindung vorliegt und R¹ nicht vorliegt; R² H und OH, H und C₁-C₆-Alkoxy oder H und C₁-C₆-Acyloxy darstellt, wobei diese Reste an den übrigen Teil des Moleküls mit Einfachbindungen gebunden sind, oder Oxo oder eine durch eine Doppelbindung gebundene, gegebenenfalls O-substituierte Oximinogruppe darstellt; R³ H und OH, H und C₁-C₆-Alkoxy oder H und C₁-C₆-Acyloxy darstellt, wobei diese Reste an den übrigen Teil des Moleküls mit Einfachbindungen gebunden sind, oder Oxo oder eine durch eine Doppelbindung gebundene, gegebenenfalls O-substituierte Oximinogruppe darstellt oder in Formel (I) oder (II) R² wie vorstehend definiert ist und R³ H, Amino, wobei die Aminogruppe gegebenenfalls mit einer C₁-C₈-Alkyl-, Aralkyl- oder Acylgruppe substituiert ist oder eine C₃-C₈-Alkenyl- oder Alkinylgruppe darstellt.

2. Verbindung nach Anspruch 1, worin R eine C₃-C₈-Cycloalkylgruppe oder eine 5- oder 6-gliedrige, Sauerstoff-oder Schwefel-enthaltende heterocyclische Gruppe darstellt.

3. Verbindung nach Anspruch 2, worin R eine Cyclohexyl-, Cyclobutyl- oder 3-Thienylgruppe darstellt.

4. Verbindung nach Anspruch 1, 2 oder 3, worin die gegebenenfalls vorliegende Bindung vorliegt; oder die gegebenenfalls vorliegende Bindung nicht vorliegt und R¹ H oder OH oder Oximino darstellt.

5. Verbindung nach einem vorangehenden Anspruch, worin R² H, OH, Oxo oder Oximino darstellt.

6. Verbindung nach einem vorangehenden Anspruch, worin R³ H, OH oder H, NHR⁸, worin R⁸ H, C₁-C₈-Alkyl oder Acyl bedeutet, darstellt.

7. Verfahren zur Herstellung einer Verbindung nach einem der Ansprüche 1 bis 6 oder einer dazu umwandelbaren Verbindung, umfassend Fermentieren von Mikroorganismus Streptomyces avermitilis ATCC 55372 in Gegenwart einer Carbonsäure der Formel RCO₂H oder eines Salzes, Esters oder Amids davon, wobei R wie in Anspruch 1 definiert ist, zur Herstellung (a) einer Verbindung der Formel (I), worin R² und R³ beide H, OH bedeuten und entweder die Doppelbindung in der 22-23-Stellung vorliegt und R¹ nicht vorliegt oder die Doppelbindung nicht vorliegt und R¹ OH darstellt oder (b) der Verbindung der Formel (II), worin R² entweder H, OH oder =O darstellt, R³ H, OH bedeutet und entweder die Doppelbindung in der 22-23-Stellung vorliegt und R¹ nicht vorliegt oder die Doppelbindung nicht vorliegt und R¹ OH darstellt und Isolieren der hergestellten Verbindung der Formel (I) oder (II), falls erforderlich, gefolgt von einem oder mehreren der nachstehenden Schritte:
(i) Hydrolysieren einer Verbindung der Formel (I) zu einer Verbindung der Formel (II) oder (III) oder Hydrolysieren einer Verbindung der Formel (II) zu einer Verbindung der Formel (III);
(ii) Umwandeln von H, OH an R² in einer Verbindung der Formel (I), (II) oder (III) durch Reaktion davon mit einem Methylhalogenid in Gegenwart von Silberoxid oder einem Silbersalz zu H, OCH₃;
(iii) Oxidieren einer Verbindung der Formel (I), (II) oder (III), worin R² oder R³ H, OH darstellt und/oder R¹ OH darstellt, zu einer Verbindung, worin mindestens einer der Reste R¹ und R² Oxo darstellt;
(iv) Umsetzen einer aus (iii) erhaltenen Verbindung mit gegebenenfalls einem O-substituierten Hydroxylamin zur Herstellung einer gegebenenfalls substituierten Iminoverbindung;
(v) Umwandeln einer Oxogruppe an R³ einer aus (iii) erhaltenen Verbindung zu einer Aminogruppe durch reduktive Aminierung unter Bereitstellung einer Verbindung der Formel (I), (II) oder (III);
(vi) Umsetzen einer Verbindung, worin R¹ OH darstellt, mit einem Alkylbromid oder -jodid in Gegenwart von Silberoxid oder einem Silbersalz zur Herstellung einer Verbindung, worin R¹ -O-Alkyl darstellt;
(vii) Reduzieren einer Verbindung der Formel (I), (II) oder (III), die an der 22-23-Stellung eine Doppelbindung aufweist, mit Wasserstoff zu einer Verbindung mit einer Einfachbindung an der 22-23-Stellung.

8. Verbindung nach einem der Ansprüche 1 bis 6, zur Verwendung in der Human- oder Veterinärmedizin.

9. Verwendung einer Verbindung nach einem der Ansprüche 1 bis 6, bei der Herstellung eines Arzneimittels zur Behandlung oder Prophylaxe von parasitischen Infektionen.

10. Streptomyces avermitilis, ATCC 55372.

## Revendications

1. Composé de formule (I), (II) ou (III) : 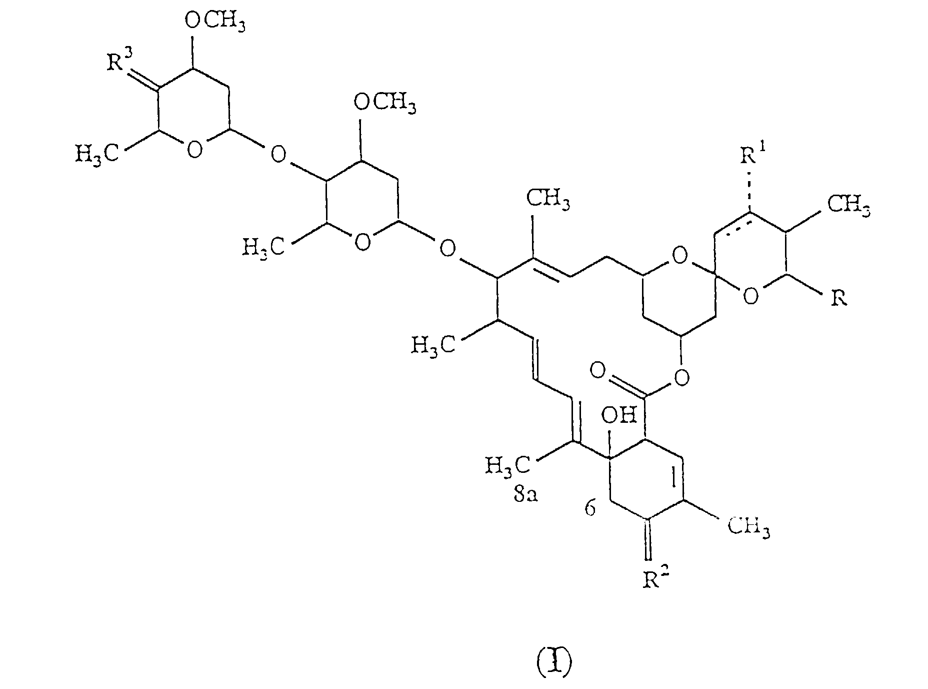 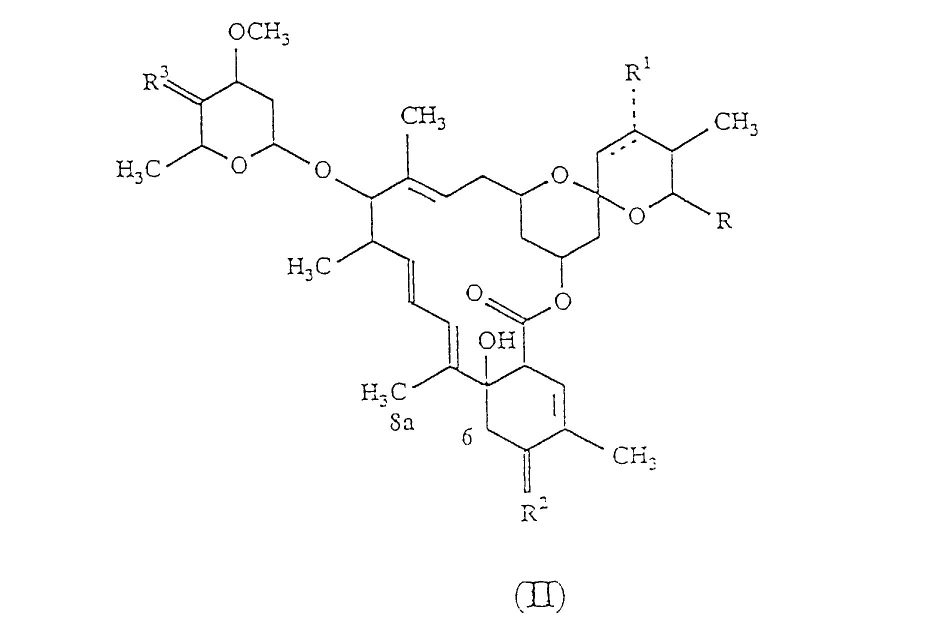 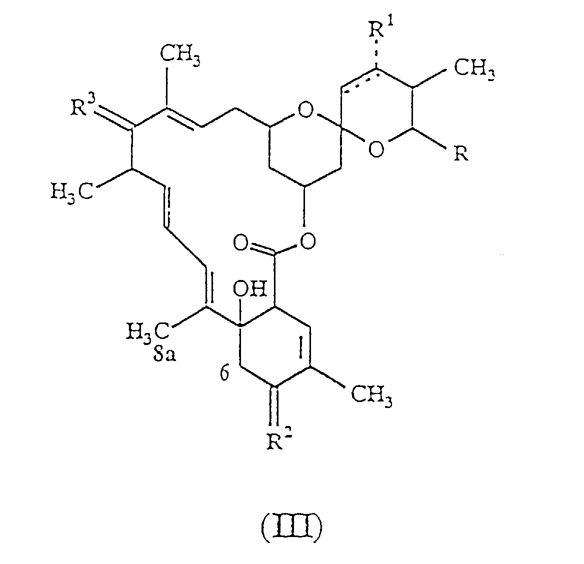 formules dans lesquelles R représente un groupe cycloalkyle en C₃-C₈ qui peut facultativement être substitué par un groupe méthylène ou par un ou plusieurs groupes alkyle en C₁-C₄ ou atomes d'halogène ; ou un noyau hétérocyclique ayant de 3 à 6 chaînons et renfermant de l'oxygène ou du soufre, lequel noyau peut être saturé, ou totalement ou partiellement insaturé et qui peut facultativement être substitué par un ou plusieurs groupes alkyle en C₁-C₄ ou atomes d'halogène ; la ligne en pointillé en position 22-23 représente une double liaison facultative, R¹ représente H, OH, O(alkyle en C₁-C₅)oxo ou oximino facultativement substitué lorsque la double liaison est absente, ou la double liaison est présente et R¹ est absent ; R² représente H et OH, H et alcoxy en C₁-C₆ ou H et acyloxy en C₁-C₆, ces radicaux étant fixés au reste de la molécule par des liaisons simples, ou représente oxo ou oximino fixé par une double liaison et facultativement O-substitué ; R³ représente H et OH, H et alcoxy en C₁-C₆ ou H et acyloxy en C₁-C₆, ces radicaux étant fixés au reste de la molécule par des liaisons simples, ou représente oxo ou oximino fixé par une double liaison et facultativement O-substitué ; ou dans la formule (I) ou (II), R² est tel que défini ci-dessus et R³ représente H, amino facultativement substitué par un groupe alkyle, aralkyle ou acyle en C₁-C₈ ou par un groupe alkényle ou alkynyle en C₃-C₈.

2. Composé selon la revendication 1, dans lequel R est un groupe cycloalkyle en C₃-C₈ ou un groupe hétérocyclique ayant 5 ou 6 chaînons et contenant de l'oxygène ou du soufre.

3. Composé selon la revendication 2, dans lequel R est un groupe cyclohexyle, cyclobutyle ou 3-thiényle.

4. Composé selon la revendication 1, 2 ou 3, dans lequel ladite liaison facultative est présente ou ladite liaison facultative est absente et R¹ représente H ou OH ou oximino.

5. Composé selon l'une quelconque des revendications précédentes, dans lequel R² représente H, OH, oxo ou oximino.

6. Composé selon l'une quelconque des revendications précédentes, dans lequel R³ représente H, OH ou H, NHR⁸ où R⁸ représente H, alkyle ou acyle en C₁-C₈.

7. Procédé de préparation d'un composé selon l'une quelconque des revendications 1 à 6 ou d'un composé transformable en un tel composé, qui consiste à faire fermenter le micro-organisme Streptomyces avermitilis ATCC 55372 en présence d'un acide carboxylique de formule RCO₂H ou d'un sel, d'un ester ou d'un amide d'un tel acide où R est tel que défini dans la revendication 1 pour produire (a) un composé de formule (I) dans lequel R² et R³ représentent tous deux H, OH et soit la double liaison en position 22-23 est présente auquel cas R¹ est absent, soit la double liaison est absente et R¹ représente OH ; ou (b) le composé de formule (II) dans lequel R² représente soit H, soit OH, soit =O, R³ représente H, OH et soit la double liaison en position 22-23 est présente auquel cas R¹ est absent, soit la double liaison est absente et R¹ représente OH, et à isoler le composé de formule (I) ou (II) produit ; opérations suivies si nécessaire par une ou plusieurs des étapes suivantes :
(i) l'hydrolyse d'un composé de formule (I) pour donner un composé de formule (II) ou (III), où l'hydrolyse d'un composé de formule (II) pour donner un composé de formule (III) ;
(ii) la transformation de H, OH au niveau R² en H, OCH₃ dans un composé de formule (I), (II) ou (III) par réaction de celui-ci avec un halogénure de méthyle en présence d'oxyde d'argent ou d'un sel d'argent ;
(iii) l'oxydation d'un composé de formule (I), (II) ou (III) dans lequel R² ou R³ représente H, OH et/ou R¹ représente OH pour donner un composé dans lequel l'un au moins de R¹ et R² représente oxo ;
(iv) la réaction d'un composé issu de l'étape (iii) avec de l'hydroxylamine facultativement O-substituée pour produire un composé imino facultativement substitué ;
(v) la transformation d'un groupe oxo au niveau R³ d'un composé issu de (iii) en un groupe amino pour obtenir un composé de formule (I), (II) ou (III) par amination réductrice ;
(vi) la réaction d'un composé dans lequel R¹ représente OH avec un bromure ou un iodure d'alkyle en présence d'oxyde d'argent ou d'un sel d'argent pour produire un composé dans lequel R¹ représente -O- alkyle ;
(vii) la réduction d'un composé de formule (I), (II) ou (III) ayant une double liaison en position 22-23 avec de l'hydrogène pour donner un composé ayant une liaison simple en position 22-23 ;

8. Composé selon l'une quelconque des revendications 1 à 6, pour une utilisation en médecine humaine ou vétérinaire.

9. Utilisation d'un composé selon l'une quelconque des revendications 1 à 6 dans la fabrication d'un médicament pour le traitement ou la prophylaxie d'infections à parasites.

10. Streptomyces avermitilis, ATCC 55372.
